# EUROPEAN PATENT SPECIFICATION

(11) **EP 2 468 203 B1**
(45) Date of publication and mention of the grant of the patent: **25.12.2013**
(21) Application number: 10826524.0
(22) Date of filing: 14.10.2010
(51) Int. Cl.: A61B 18/00, A61B 17/32

(54) **MEDICAL DEVICE**
MEDIZINISCHE VORRICHTUNG
DISPOSITIF MÉDICAL

(30) Priority: 28.10.2009 US 255540 P
(43) Date of publication of application: 27.06.2012
(73) Proprietor: OLYMPUS MEDICAL SYSTEMS CORP., Tokyo 151-0072 (JP)
(72) Inventor: KONISHI, Sumihito, Tokyo 151-0072 (JP); SUGAYA, Kota, Tokyo 151-0072 (JP)
(74) Representative: Schicker, Silvia
(86) International application number: PCT/JP2010/068043
(87) International publication number: WO 2011/052390

(56) References cited:
- EP-A1- 1 707 147
- JP-A- 7 231 896
- JP-A- 7 231 896
- JP-A- 2004 235 962
- JP-A- 2008 055 151
- JP-A- 2008 176 461

## Description

### Technical Field

The present invention relates to a medical apparatus, and more particularly, to a medical apparatus provided with a display section that displays setting information of a medical device.

### Background Art

In recent years, ultrasound output apparatuses for treating a living tissue of a treatment target using ultrasound energy or high frequency output apparatuses (also referred to as "electric scalpel apparatus") that perform treatment using a high frequency current are widely used.

The ultrasound output apparatuses are provided with a display section that displays setting information of a medical device connected. Users check the setting information displayed on the display section to grasp an operation status of the medical device.

In recent years, it has been configured that a plurality of medical devices are connected to the ultrasound output apparatus and there has arisen a big problem of efficiently displaying the setting information of the plurality of medical devices on the display section.

For example, Japanese Patent Application Laid-Open Publication No. 7-231896 discloses a system control apparatus that automatically recognizes connection statuses of a plurality of apparatuses and displays set values or operation statuses of the apparatuses connected in a predetermined display region of a display screen.

This system control apparatus is configured such that a system controller detects connection statuses of a plurality of apparatuses such as light source apparatus and insufflator and displays set values of the apparatuses connected using a 7-segment LED in a predetermined display region of a concentrated display panel.

However, in a case of allowing a plurality of apparatuses to be connected, setting information to be displayed on the display screen is increased in accordance with the number of apparatuses. Further, since the size of the display screen is limited, a display region for displaying the setting information of one apparatus is decreased as the number of connected apparatuses is increased. Therefore, if it is configured to always display the setting information of all the connectable apparatuses, there is a problem that recognizability of the setting information for a user is deteriorated.

JP 7231896 discloses a system controller connected to several devices and controls. Each device is connected to a concentrated display panel for displaying the condition of each device and a concentrated operation panel.

EP 1 707 147 A1 discloses an electrosurgical instrument. An operating mode as well as other operating parameters, especially a power level, are preset by a surgeon before starting a procedure. A non-volatile memory stores the set operating parameters. Several operating modes are available using a three-button fingerswitch handpiece and a 3-way footswitch.

In view of the above, an object of the present invention is to provide a medical apparatus capable of automatically displaying an optimal screen image by dividing a display screen in accordance with connection state of medical devices.

### Disclosure of Invention

### Means for Solving the Problem

The invention is defined in claim 1. According to one aspect of the present invention, a medical apparatus to which at least two medical devices are connectable, comprises: a display section for displaying setting information of the respective devices provided with a first display screen that displays setting information of a first medical device and a second display screen that displays setting information of a second medical device; and a control section that controls a number of partitions of the display section according to connection statuses of the first and second medical devices so as to display, when it is detected that the first medical device and the second medical device are connected to the medical apparatus, setting information of the first and second medical devices on the first and second display screens, respectively, display, when it is detected that only the first medical device is connected to the medical apparatus, the setting information of the first medical device on the entire display section and display, when it is detected that only the second medical device is connected to the medical apparatus, the setting information of the second medical device on the entire display section.

### Brief Description of the Drawings

Fig. 1 is a diagram illustrating a configuration of a surgery system according to an embodiment of the present invention;
Fig. 2 is a front view of an ultrasound output apparatus viewed from the front;
Fig. 3A is a block diagram illustrating an internal configuration of a high frequency output apparatus;
Fig. 3B is a block diagram illustrating an internal configuration of the ultrasound output apparatus;
Fig. 4 is a diagram illustrating duplexing of output of the output circuit 44;
Fig. 5 is a diagram illustrating an electric configuration of a handpiece connector and an output connector;
Fig. 6 is a diagram illustrating a display example of a menu screen 70;
Fig. 7 is a diagram illustrating an example of display screen when a handpiece 2a is connected to the ultrasound output apparatus 4;
Fig. 8 is a diagram illustrating an example of display screen when a handpiece 2b is connected to the ultrasound output apparatus 4;
Fig. 9 is a diagram illustrating an example of display screen when the handpieces 2a and 2b are connected to the ultrasound output apparatus 4;
Fig. 10 is a diagram illustrating an example of display screen when the handpieces 2a and 2b are not connected to the ultrasound output apparatus 4;
Fig. 11 is a diagram illustrating an example of display screen when the handpiece 2b is not connected to the ultrasound output apparatus 4;
Fig. 12 is a diagram illustrating an example of display screen in the case of output in a SEAL & CUT mode when the handpiece 2a is connected to the ultrasound output apparatus 4;
Fig. 13 is a diagram illustrating an example of display screen in the case of output in a SEAL mode when the handpiece 2a is connected to the ultrasound output apparatus 4;
Fig. 14 is a diagram illustrating an example of display screen in the case of output in a MAX mode when the handpiece 2b is connected to the ultrasound output apparatus 4;
Fig. 15 is a diagram illustrating an example of display screen in the case of output in a VAR mode when the handpiece 2b is connected to the ultrasound output apparatus 4;
Fig. 16 is a diagram illustrating an example of display screen in the case of output in a SEAL & CUT mode while the handpieces 2a and 2b are connected to the ultrasound output apparatus 4;
Fig. 17 is a diagram illustrating an example of display screen in the case of output in a SEAL mode while the handpieces 2a and 2b are connected to the ultrasound output apparatus 4;
Fig. 18 is a diagram illustrating an example of display screen in the case of output in a MAX mode while the handpieces 2a and 2b are connected to the ultrasound output apparatus 4;
Fig. 19 is a diagram illustrating an example of display screen in the case of output in a VAR mode while the handpieces 2a and 2b are connected to the ultrasound output apparatus 4;
Fig. 20 is a diagram illustrating an example of display screen in the case where three handpieces are connected to the ultrasound output apparatus 4;
Fig. 21 is a diagram illustrating an example of display screen in the case where four handpieces are connected to the ultrasound output apparatus 4;
Fig. 22 is a flowchart illustrating an example of flow of display processing;
Fig. 23 is a flowchart illustrating an example of flow of change processing; and
Fig. 24 is a flowchart illustrating an example of flow of highlighting processing.

### Best Mode for Carrying Out the Invention

Hereinafter, an embodiment of the present invention will be described in detail with reference to the accompanying drawings.

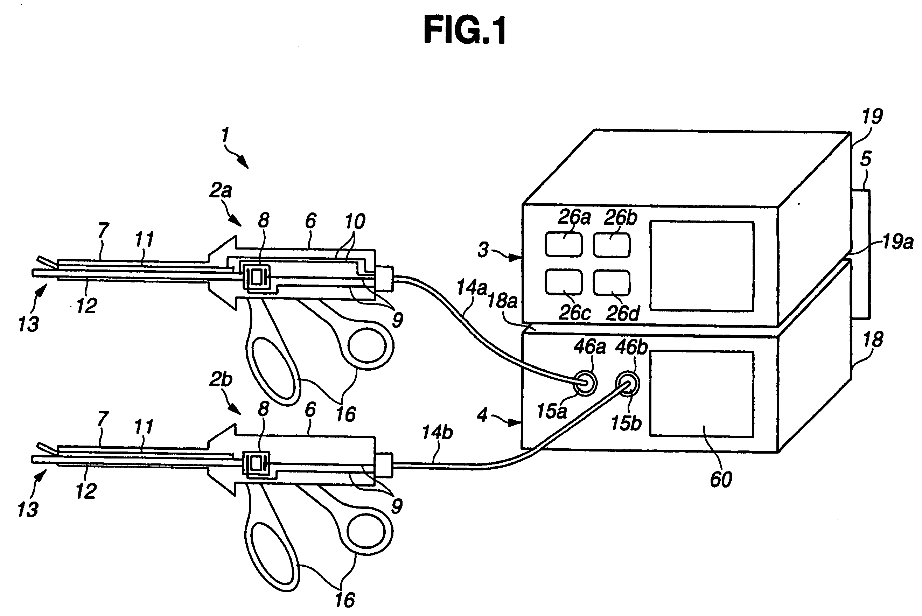

As shown in Fig. 1, a surgery system 1 of an embodiment of the present invention is configured by including handpieces 2a and 2b, a high frequency output apparatus 3 and an ultrasound output apparatus 4.

The high frequency output apparatus 3 and the ultrasound output apparatus 4 are connected together via a docking connector which will be described later. Furthermore, the high frequency output apparatus 3 and the ultrasound output apparatus 4 are connected together on the rear side via, for example, communication cable 5.

The high frequency output apparatus 3 outputs a high frequency signal to the handpiece 2a via the docking connector and the ultrasound output apparatus 4.

The ultrasound output apparatus 4 as a medical apparatus outputs an ultrasound drive signal that causes ultrasound transducers 8, which will be described later, incorporated in the handpieces 2a and 2b to vibrate by means of ultrasound.

The handpieces 2a and 2b are medical devices that perform treatment of a living tissue which is a treatment target. Especially, the handpiece 2a is a medical device that simultaneously outputs an ultrasonic wave and a high frequency to perform treatment of a living tissue which is a treatment target and the handpiece 2b is a medical device that outputs only ultrasound to perform treatment of a living tissue which is a treatment target.

The handpiece 2a has two output modes; a coagulation dissection mode (hereinafter, also referred to as "SEAL & CUT mode") in which ultrasound and high frequency are outputted simultaneously and a coagulation mode (hereinafter, also referred to as "SEAL mode") in which only high frequency is outputted. These two modes are switched by a hand switch (not shown) provided in a grasping portion 6 of the handpiece 2a, which will be described later.

Furthermore, the handpiece 2b has two output modes; a maximum output mode (hereinafter, also referred to as "MAX mode") in which ultrasound is outputted at a maximum level and a variable output mode (hereinafter, also referred to as "VAR mode") in which ultrasound is outputted at a variable level. These two modes are switched by a hand switch (not shown) provided in a grasping portion 6 of the handpiece 2b, which will be described later.

The handpiece 2a has the grasping portion 6 grasped and operated by an operator and a sheath portion 7 extending forward from the grasping portion 6. The grasping portion 6 and the sheath portion 7 incorporate the ultrasound transducer 8, an ultrasound cable 9, a high frequency cable 10, a wire 11 and a probe 12.

On the distal end side of the probe 12, a treatment portion 13 is formed of a distal end portion of the probe 12 and a movable piece which freely opens/closes with respect to this distal end portion.

Furthermore, a distal end of a cable 14a is connected to a rear end of the grasping portion 6. A handpiece connector (hereinafter, referred to as "HP connector") 15a is provided at a rear end of the cable 14a and the HP connector 15a is detachably connected to an output connector 46a of the ultrasound output apparatus 4.

The ultrasound cable 9 and the high frequency cable 10 are passed through the cable 14a and rear ends of the ultrasound cable 9 and the high frequency cable 10 are connected to the output connector 46a of the ultrasound output apparatus 4 via the HP connector 15a.

The ultrasound output apparatus 4 is enabled to supply an ultrasound drive signal to the ultrasound transducer 8 inside the grasping portion 6 via the ultrasound cable 9 in the cable 14a. Supplying the ultrasound drive signal causes the ultrasound transducer 8 to perform ultrasonic vibration. The ultrasonic vibration of the ultrasound transducer 8 is transmitted to the distal end portion via the probe 12 inside the sheath portion 7. With this ultrasound vibration energy, the handpiece 2a can generate frictional heat in the living tissue of the treatment target and perform treatment such as coagulation and dissection.

Furthermore, the two high frequency cables 10 for transmitting a high frequency signal are also passed through the cable 14a, one distal end of the high frequency cable 10 is connected to the rear end of the probe 12 and the other distal end is connected to the rear end side of the wire 11 which is electrically continuous with the movable piece. The movable piece, the probe 12 and the wire 11 are formed of a conductor such as metal that transmits a high frequency signal. The high frequency cable 10 may be configured to be connected not to the rear end side of the wire 11, but to the movable piece via a lead wire passed through the sheath portion 7. By passing a high frequency current through the living tissue grasped by the treatment portion 13, the handpiece 2a can perform treatment of high frequency cauterization.

Furthermore, the grasping portion 6 is provided with finger hooking parts 16 for performing an opening/closing operation. The operator hooks the fingers to the finger hooking parts 16 to perform an opening/closing operation, pulls the wire 11 inserted in the sheath portion 7, opens/closes the movable piece in the treatment portion 13, and can thereby grasp the living tissue of the treatment target.

On the other hand, since the handpiece 2b outputs only ultrasound, the handpiece 2b has no high frequency cable 10 unlike the handpiece 2a. The rest of the configuration is similar to that of the handpiece 2a, and therefore detailed descriptions thereof will be omitted.

A distal end of a cable 14b is connected to a rear end of a grasping portion 6 of the handpiece 2b. An HP connector 15b at a rear end of the cable 14b is detachably connected to the output connector 46b of the ultrasound output apparatus 4.

An ultrasound cable 9 is passed through the cable 14b and a rear end of the ultrasound cable 9 is connected to the output connector 46b of the ultrasound output apparatus 4 via the HP connector 15b.

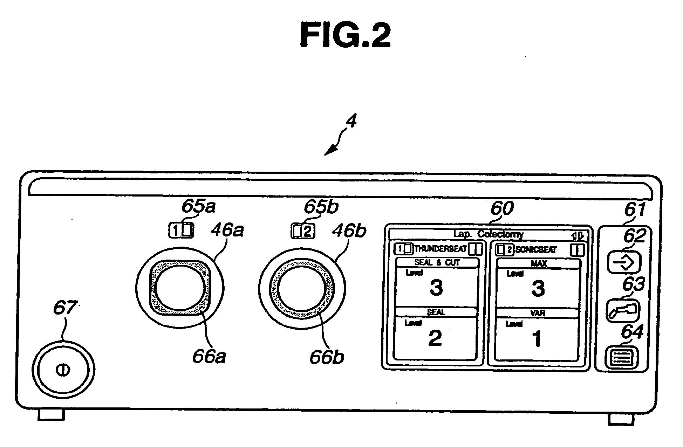

Next, a configuration of a front side of the ultrasound output apparatus 4 will be described using Fig. 2. Fig. 2 is a front view of the ultrasound output apparatus 4 viewed from the front.

As shown in Fig. 2, the ultrasound output apparatus 4 is provided with output connectors 46a and 46b, a display section 60, a sheet switch 61, symbols 65a and 65b and a power supply button 67. Furthermore, the sheet switch 61 is provided with a selection button 62, a probe check button 63 and a menu button 64, and the output connectors 46a and 46b are provided with flange receivers 66a and 66b respectively.

The display section 60 displays an individual device setting screen or the like, which will be described later. The display section 60 is provided with a touch panel on a liquid crystal display and the user is enabled to change a setting of output level or the like by touching the display screen by his/her finger or a special pen.

The probe check button 63 is a button to make a probe check. As described above, since the handpiece 2a simultaneously outputs high frequency and ultrasound, it is necessary to make a probe check by interposing a conductive substance between the movable piece and the probe 12. According to the present embodiment, the probe check button 63 is used to make a probe check by only outputting ultrasound. This eliminates the necessity for interposing a conductive substance between the movable piece and the probe 12 to make a check.

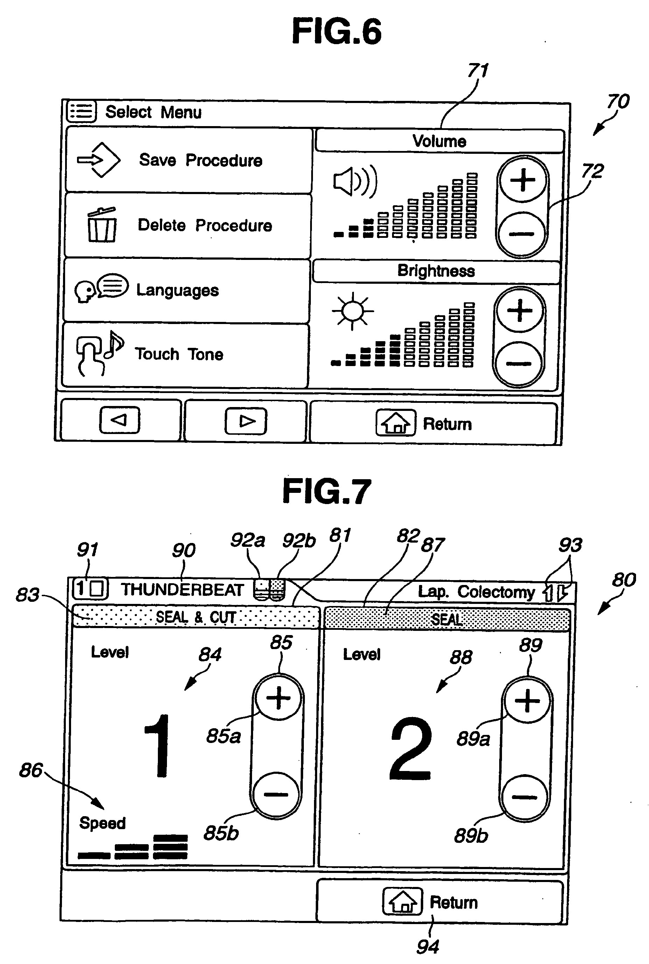

The menu button 64 is a button for displaying a menu screen 70 shown in Fig. 6. By pressing the menu button 64, the user can cause the menu screen 70 to be displayed on the display section 60.

The symbols 65a and 65b are provided so as to correspond to the output connectors 46a and 46b on the front of the ultrasound output apparatus 4. A symbol similar to the symbol 65a is provided, for example, in the grasping portion 6 of the handpiece 2a by means of printing or the like. Moreover, a symbol similar to the symbol 65b is provided, for example, in the grasping portion 6 of the handpiece 2b by means of printing or the like. This allows the operator to check symbols provided in the grasping portion 6 of the handpiece 2a and recognize to which of the output connectors 46a and 46b the HP connector 15a should be connected. As a result, it is possible to prevent the operator from erroneously connecting the HP connector 15a to the output connector 46b.

Furthermore, a plurality of (here, two) output connectors 46a and 46b are provided in the ultrasound output apparatus 4. When the two output connectors 46a and 46b have the same shape, providing the aforementioned symbols 65a and 65b alone may not prevent misconnections of the HP connectors.

Thus, the HP connector 15a is provided with a quasi-rectangular flange (not shown) and this quasi-rectangular flange is designed to engage with the flange receiver 66a of the output connector 46a. On the other hand, the HP connector 15b is provided with a quasi-circular flange (not shown) and this quasi-circular flange is designed to engage with the flange receiver 66b of the output connector 46b. This can prevent misconnections between the HP connectors and the output connectors.

The shapes of the flange receivers 66a and 66b may be made more distinctive by coloring the flange receivers 66a and 66b in different colors.

The power supply button 67 is a button to turn ON or OFF the power of the ultrasound output apparatus 4.

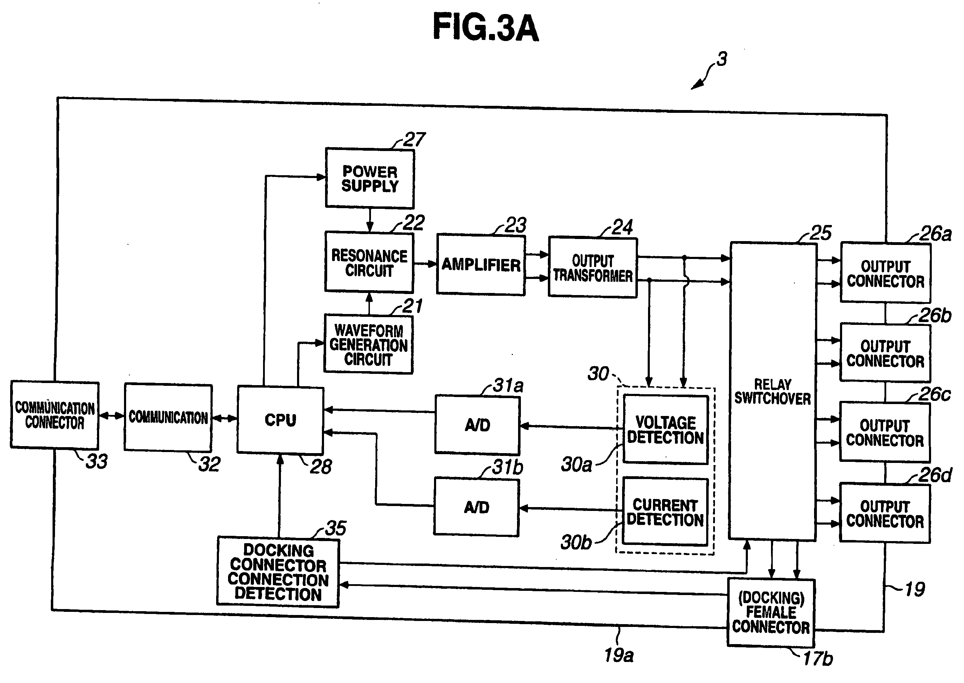

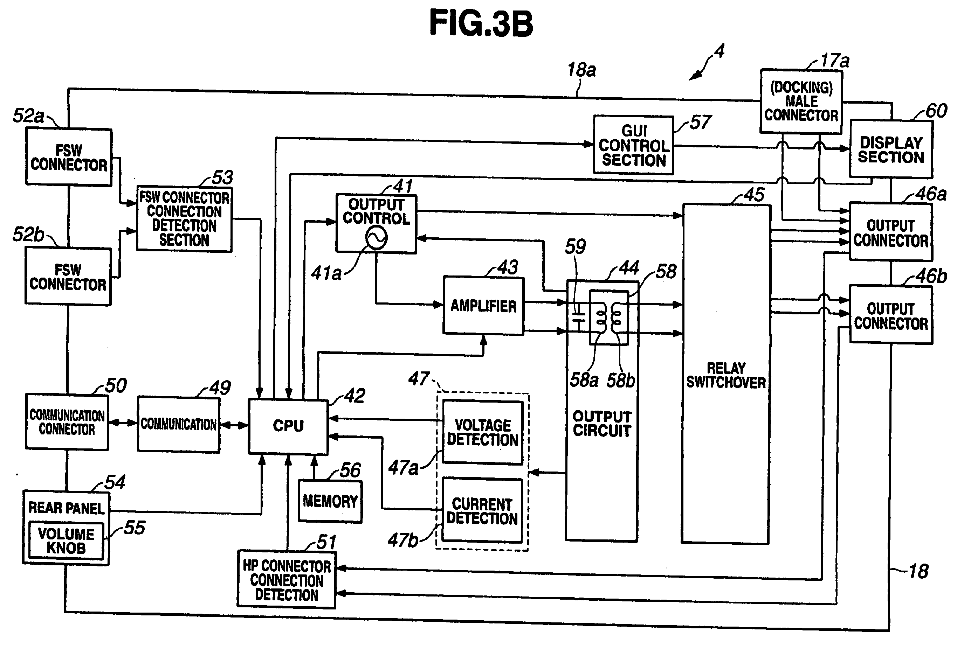

Here, internal configurations of the high frequency output apparatus 3 and the ultrasound output apparatus 4 will be described using Fig. 3A and Fig. 3B.

Fig. 3A is a block diagram illustrating an internal configuration of the high frequency output apparatus 3 and Fig. 3B is a block diagram illustrating an internal configuration of the ultrasound output apparatus 4.

First, a configuration of the docking connector will be described.

A top plate 18a of a housing 18 as a housing case of the ultrasound output apparatus 4 is provided with a docking male connector (abbreviated as "male connector") 17a making up a docking connector.

On the other hand, a bottom plate 19a of a housing 19 as a housing case of the high frequency output apparatus 3 is provided with a docking female connector (abbreviated as "female connector") 17b making up the docking connector. By placing the housing 19 of the high frequency output apparatus 3 on the top plate 18a of the housing 18 of the ultrasound output apparatus 4, it is possible to cause the male connector 17a and the female connector 17b provided at positions facing each other on both plate surfaces to dock with each other and make both connectors connected.

A high frequency signal outputted from the high frequency output apparatus 3 passes from the female connector 17b provided on the bottom plate 19a of the housing 19 through the male connector 17a provided at a position on the top plate 18a of the housing 18 of the ultrasound output apparatus 4 opposed thereto and is outputted to the output connector 46a. The high frequency signal is then transmitted to the handpiece 2a via the cable 14a connected to the HP connector 15a connected to the output connector 46a.

Next, the internal configuration of the high frequency output apparatus 3 will be described. The high frequency output apparatus 3 incorporates a waveform generation circuit 21 for generating a sine wave or burst wave and the sine wave or burst wave signal outputted from this waveform generation circuit 21 is inputted to the amplifier 23 via a resonance circuit 22.

A signal amplified by an amplifier 23 is applied to a primary wiring side of an output transformer 24 and a high frequency signal for cauterization is generated on the secondary wiring side.

The secondary wiring of this output transformer 24 is connected to, for example, four output connector 26a, 26b, 26c and 26d, and the female connector 17b making up the docking connector via a relay switchover circuit 25 that switches between high frequency signals outputted.

As described above, the female connector 17b is provided on the bottom plate 19a of the housing 19. Furthermore, the resonance circuit 22 receives a power supply voltage from a variable voltage power supply circuit 27, and the waveform generation circuit 21 and the power supply circuit 27 are controlled by a CPU 28 as a control section.

The CPU 28 controls the waveform generation circuit 21 and the power supply circuit 27 in accordance with a setting of the output mode by a setting section (not shown) and an output set value or the like.

The output signal of the secondary wiring of the aforementioned output transformer 24 is inputted to a voltage detection circuit 30a and a current detection circuit 30b making up a detection section 30.

The voltage detection circuit 30a and the current detection circuit 30b measure, in other words, detect the voltage and current of a high frequency signal outputted from the secondary wiring of the output transformer 24 respectively. The detected voltage and current are converted to a digital voltage and current by A/D convertors 31a and 31b respectively and inputted to the CPU 28.

The CPU 28 calculates, in other words, detects high frequency power as the product of the voltage and current inputted. The CPU 28 controls the voltage of the power supply circuit 27 so that the value of the detected high frequency power becomes a set value predetermined by the aforementioned setting section.

The CPU 28 is connected to a communication connector 33 via a communication circuit 32 that performs communication. The communication connector 33 is connected to a communication connector 50 of the ultrasound output apparatus 4 shown in Fig. 3B via the communication cable 5.

The female connector 17b connected to the relay switchover circuit 25 is detachably connected to the male connector 17a of the ultrasound output apparatus 4 as described above.

Furthermore, for example, two connector pins for connection detection of the female connector 17b are connected to a docking connector connection detection circuit 35 and this docking connector connection detection circuit 35 detects connection between the male connector 17a and the female connector 17b using the connector pins for connection detection all the time.

In this case, the two connector pins for connection detection are set so as to be connected with, for example, the two short-circuited connector pins of the other male connector 17a.

Therefore, the docking connector connection detection circuit 35 detects whether or not the two connector pins for connection detection are electrically continuous, and can thereby detect whether or not the docking connector 17 is connected.

The connection detection result by the docking connector connection detection circuit 35 is transmitted to the CPU 28. When the connection detection result by the docking connector connection detection circuit 35 shows that the docking connector 17 is not connected, the CPU 28 prohibits simultaneous outputs of the ultrasound output and the high frequency output.

In other words, the CPU 28 allows simultaneous outputs of the ultrasound output and the high frequency output only when the connection of the docking connector 17 is detected.

On the other hand, when the docking connector connection detection circuit 35 detects that the connection between the male connector 17a and the female connector 17b, the docking connector connection detection circuit 35 controls the switchover of the relay switchover circuit 25 to perform switching so that the output signal of the output transformer 24 is outputted to the female connector 17b side. Instead of the docking connector connection detection circuit 35, the CPU 28 may control the switching.

On the other hand, the ultrasound output apparatus 4 shown in Fig. 3B has an output control circuit 41 which incorporates an oscillating circuit 41a. This output control circuit 41 adjusts the frequency and current of an oscillating signal oscillated by this oscillating circuit 41a under the control of a CPU 42 as the control section and outputs the adjusted signal to an amplifier 43.

The signal amplified by the amplifier 43 is inputted to an output circuit 44 and applied to a primary wiring 58a of a transformer 58 of the output circuit 44. The voltage of the signal amplified by the amplifier 43 is amplified by the transformer 58 and outputted from a secondary wiring 58b of the transformer 58 as an ultrasound drive signal.

In the present embodiment, a capacitor 59 is provided on the input side of the transformer 58. To be more specific, the capacitor 59 is provided parallel to the primary wiring 58a side of the transformer 58. The capacitor 59 and the primary wiring 58a constitute an LC filter as a noise reduction filter.

According to such a configuration, the noise reduction filter is configured using the primary wiring 58a of the transformer 58 mounted for a different function, and therefore the LC filter can be formed only by adding the capacitor 59. Thus, the number of parts can be reduced compared to a case where an LC filter is newly provided.

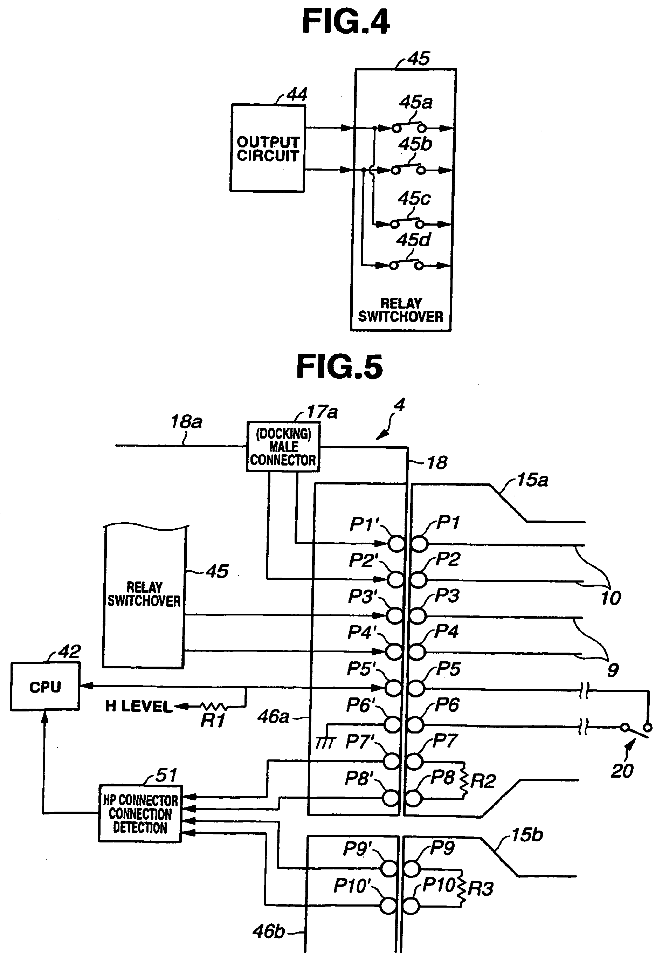

The ultrasound drive signal outputted from the transformer 58 is connected to the two output connectors 46a and 46b via a relay switchover circuit 45 that switches and outputs this signal. The gain of the amplifier 43 is controlled by the CPU 42. Thus, since outputs to the output connectors 46a and 46b are switched by the relay switchover circuit 45, the output of the output circuit 44 is duplexed and outputted to the output connectors 46a and 46b as shown in Fig. 4 to prevent erroneous switching to the output connectors 46a and 46b.

Fig. 4 illustrates the duplexing of the output of the output circuit 44.

The relay switchover circuit 45 has switches 45a to 45d, the switch 45a is H active, the switch 45b is L active, the switch 45c is H active and the switch 45d is L active. Therefore, when H as switching signals of the switches 45a and 45c, L as switching signals of the switches 45b and 45d are not inputted, ultrasound drive signals to the output connectors 46a and 46b are not outputted.

Furthermore, the amplifier 43 of the present embodiment is made up of a digital amplifier. This digital amplifier performs pulse width modulation (PWM) on an analog input signal using a carrier.

This pulse width modulation wave is switched by a switching circuit inside the digital amplifier and then passed through a low pass filter (LPF) and outputted to the output circuit 44 as an ultrasound drive signal which is the amplified input signal.

Adopting such a digital amplifier makes it possible to generate an ultrasound drive signal efficiently.

In the present embodiment, since the frequency of the ultrasound drive signal is on the order of 47 kHz which is a higher frequency than an audible frequency domain, a carrier with a higher frequency (e.g., 1 MHz) than the carrier frequency used for an audio device is used.

The output connector 46a is also connected to the male connector 17a. The connector 46a is connected to the handpiece 2a that outputs an ultrasonic wave and a high frequency.

The output connector 46b is not connected to the male connector 17a but to the ultrasound-specific handpiece 2b that outputs an ultrasound wave independently of the high frequency output apparatus 3.

The ultrasound drive signal outputted from the output circuit 44 is inputted to a voltage detection circuit 47a and a current detection circuit 47b making up a detection section 47 to measure, in other words, detect the voltage and current respectively.

The detected voltage and current are inputted to the CPU 42 via an A/D convertor (not shown) in the voltage detection circuit 47a and the current detection circuit 47b.

Furthermore, the ultrasound output apparatus 4 is provided with a setting section (not shown) that sets power of an ultrasound drive signal to be supplied to the ultrasound transducer 8 of the handpiece 2a and setting information thereof is inputted to the CPU 42.

The CPU 42 performs constant current control via the output control circuit 41 based on the voltage and current detected via the detection section 47 so as to output the power set by the setting section from the output circuit 44.

For this reason, the control information of the output value when outputted from the output circuit 44 is temporarily stored in the memory in the output control circuit 41 and the CPU 42 performs control so as to correct immediately preceding control information via the output control circuit 41 according to the voltage and current detected thereafter.

Furthermore, the CPU 42 is connected to the communication connector 50 via a communication circuit 49 that performs communication. This communication connector 50 is connected to the communication connector 33 of the high frequency output apparatus 3 side shown in Fig. 3A via the communication cable 5. The CPU 42 and the CPU 28 can perform bidirectional communication via the communication cable 5.

Furthermore, the connector connection detection pins of the output connectors 46a and 46b are connected to an HP connector connection detection circuit 51. The HP connector connection detection circuit 51 detects connection or no connection of the HP connectors 15a and 15b.

As described above, the handpiece 2a is connected to the output connector 46a and the handpiece 2b is connected to the output connector 46b. The HP connector connection detection circuit 51 transmits the detection result information to the CPU 42.

The CPU 42 controls the switching of the relay switchover circuit 45 via the output control circuit 41 so as to supply, to the output connector to which the handpiece is connected, the output signal from the output circuit 44, that is, ultrasound drive signal according to the information of the detection result. The CPU 42 may also be configured to control the switching of the relay switchover circuit 45.

Furthermore, the ultrasound output apparatus 4 includes foot switch (hereinafter referred to as "FSW") connectors 52a and 52b to which two foot switches (not shown) are connected. The FSW connectors 52a and 52b are connected to an FSW connector connection detection section 53.

The FSW connector connection detection section 53 detects whether or not the foot switches are connected to the FSW connectors 52a and 52b respectively and outputs the detection information to the CPU 42.

Furthermore, a rear panel 54 is provided on the rear face of the ultrasound output apparatus 4. The rear panel 54 is provided with a volume knob 55 and the user can adjust the volume of the ultrasound output apparatus 4 by operating the volume knob 55. Pressing the menu button 64 causes the menu screen 70 shown in Fig. 6 to be displayed on the display section 60. The operator can adjust the volume of the ultrasound output apparatus 4 by operating a volume control button 72 of a volume setting section 71 of the menu screen 70. The volume set by the volume setting section 71 of the menu screen 70 is linked with the volume knob 55 of the rear panel 54. Furthermore, the volume may also be linked with a volume knob (not shown) of the high frequency output apparatus 3 and this allows the volume of the high frequency output apparatus 3 to match the volume of the ultrasound output apparatus 4.

A memory 56 stores setting information corresponding to the previous usage, that is, information of the output level or the like. The CPU 42 reads information of the output level or the like from the memory 56 and outputs the information to a GUI control section 57.

The GUI control section 57 performs control of automatically switching a display screen displayed on the display section 60 according to the connection statuses of the handpieces 2a and 2b and also performs control of causing the display section 60 to display information of the output level or the like.

Fig. 5 is a diagram illustrating an electric configuration of the handpiece connector and the output connector. Connector pins P1 and P2 are connected to the male connector 17a via connectors pins P1' and P2' of the output connector 46a respectively. Connector pins P3 and P4 are connected to the relay switchover circuit 45 via connector pins P3' and P4' of the output connector 46a respectively.

Furthermore, connector pins P5 and P6 connected to an output switch 20 provided in the handpiece 2 are connected to connector pins P5' and P6' on the output connector 46b side respectively.

In the example of Fig. 5, the connector pin P6' is grounded and the connector pin P5' is connected to the CPU 42. In this case, the connector pin P5' is pulled up to H level, for example, by a resistor R1. When the output switch 20 is turned ON, the level of the connector pin P5' changes from H level to L level and the CPU 42 detects that the output switch 20 is turned ON. The CPU 42 transmits the signal turned ON to the CPU 28 of the high frequency output apparatus 3 via the communication cable 5 to output a high frequency signal and also outputs an ultrasound drive signal.

To be more specific, the ultrasound cable 9 is connected to the relay switchover circuit 45 shown in Fig. 3B via the output connector 46a. On the other hand, the high frequency cable 10 is electrically connected to the relay switchover circuit 25 inside the high frequency output apparatus 3 via the docking connector as a connection section between the ultrasound output apparatus 4 and the high frequency output apparatus 3 via the output connector 46a.

Turning ON the output switch 20 which performs operation of instructing simultaneous outputs of ultrasound and high frequency causes ON information of the output switch 20 to be transmitted to the CPU 28 of the high frequency output apparatus 3 from the CPU 42 of the ultrasound output apparatus 4 via the communication cable 5 and a high frequency signal and a ultrasound drive signal are simultaneously outputted to the handpiece 2a.

Connector pins P7 and P8 are connection detecting pins and connected to connector pins P7' and P8' on the output connector 46a side respectively. The connector pins P7' and P8' are connected to the HP connector connection detection circuit 51. Furthermore, a resistor R2 is provided between the connector pins P7 and P8.

The HP connector connection detection circuit 51 detects the resistance value of the resistor R2 to thereby detect whether or not the HP connector 15a is connected to the output connector 46a and also detect what type of handpiece 2a is connected based on the resistance value of the resistor R2.

Likewise, connector pins P9 and P10 which are connection detecting pins of the HP connector 15b are connected to connector pins P9' and P10' on the output connector 46b side. Furthermore, a resistor R3 is provided between the connector pins P9 and P10. Furthermore, connector pins other than the connector pins P9 and P10 which are connection detecting pins of the HP connector 15b are not shown.

The HP connector connection detection circuit 51 detects the resistance value of the resistor R3, thereby detects whether or not the HP connector 15b is connected to the output connector 46b and also detects what kind of handpiece 2b is connected based on the resistance value of the resistor R3.

Here, the control of automatically switching the display screen displayed according to the connection statuses of the handpieces 2a and 2b will be described. First, a display screen when the handpiece 2a is connected to the ultrasound output apparatus 4 will be described.

Fig. 7 is a diagram illustrating an example of display screen when the handpiece 2a is connected to the ultrasound output apparatus 4.

The display screen shown in Fig. 7 is an individual device setting screen 80 which is automatically displayed on the display section when the handpiece 2a is connected.

The individual device setting screen 80 includes a first display screen 81 that displays setting information of the SEAL & CUT mode and a second display screen 82 that displays setting information of the SEAL mode.

The first display screen 81 includes a mode information display section 83, an output level display section 84, an output level changing section 85 and a dissection speed display section 86.

"SEAL & CUT" is displayed on the mode information display section 83 in correspondence with the SEAL & CUT mode. The currently set output level is displayed on the output level display section 84. The output level of the SEAL & CUT mode is set to level 1 in Fig. 7. In the present embodiment, the output level is enabled to be changed, for example, in three stages of level 1 to level 3.

The output level changing section 85 has a + button 85a and - button 85b. The user can increase or decrease the output level by pressing the + button 85a or - button 85b. That is, the output level of the SEAL & CUT mode can be changed between the aforementioned level 1 and level 3.

The dissection speed display section 86 displays a dissection speed corresponding to the output level displayed on the output level display section 84, that is, the output level of the SEAL & CUT mode.

The second display screen 82 includes a mode information display section 87, an output level display section 88 and an output level changing section 89.

"SEAL" is displayed on the mode information display section 87 in correspondence with the SEAL mode. The current output level is displayed on the output level display section 88. In Fig. 7, the output level of the SEAL mode is set to level 2.

The output level changing section 89 has a + button 89a and a - button 89b to allow the output level of the SEAL mode to be changed between aforementioned level 1 and level 3.

Furthermore, the individual device setting screen 80 has a model name display section 90, a symbol icon 91, FSW connection detection icons 92a and 92b, a communication connection detection icon 93 and a return button 94.

The model name display section 90 displays the model name of the handpiece 2a connected to the ultrasound output apparatus 4. For example, "THUNDERBEAT" (registered trademark) is displayed on the model name display section 90 in Fig. 7 as the model name corresponding to the handpiece 2a.

The symbol icon 91 displays the same symbol as the aforementioned symbol 65a as an icon. Furthermore, this symbol is also provided in the grasping portion 6 of the handpiece 2a as described above. This allows the user to recognize that the setting information displayed on the individual device setting screen 80 as the setting information of the handpiece 2a by checking the symbol icon 91.

The FSW connection detection icons 92a and 92b are icons displayed when the FSW connector connection detection section 53 detects that the foot switches (not shown) are connected to the FSW connectors 52a and 52b respectively. Furthermore, the FSW connection detection icon 92a and the mode information display section 83 are displayed in the same color and the FSW connection detection icon 92b and the mode information display section 87 are displayed in the same color.

This allows the user to check the FSW connection detection icons 92a and 92b to thereby change the output level or the like.

The communication connection detection icon 93 is an icon displayed when communication between the high frequency output apparatus 3 and the ultrasound output apparatus 4 is established. As described above, when a treatment is performed with high frequency output, communication is performed between the high frequency output apparatus 3 and the ultrasound output apparatus 4 via the communication cable 5. Thus, when communication is not established, a treatment with high frequency output cannot be performed. Thus, when communication between the high frequency output apparatus 3 and the ultrasound output apparatus 4 is established, displaying the communication connection detection icon 93 allows the user to recognize that it is possible to perform a treatment with high frequency output.

The return button 94 is a button to return to a total device display screen which will be described later. The user can return to the total device display screen by pressing the return button 94.

Next, the display screen when the handpiece 2b is connected to the ultrasound output apparatus 4 will be described.

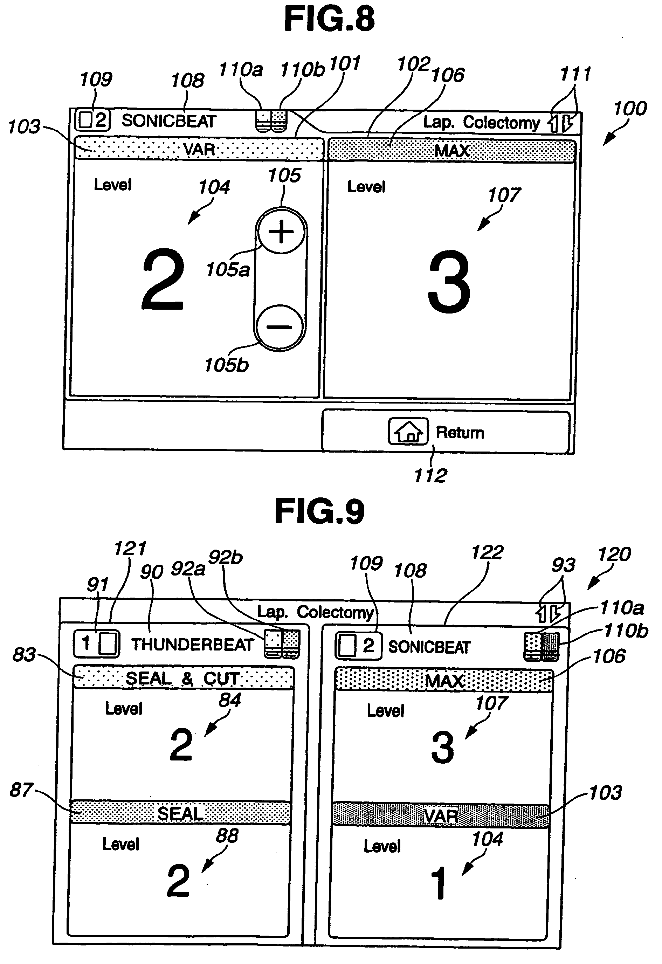

Fig. 8 is a diagram illustrating an example of display screen when the handpiece 2b is connected to the ultrasound output apparatus 4.

The display screen shown in Fig. 8 is an individual device setting screen 100 which is automatically displayed on the display section when the handpiece 2b is connected.

The individual device setting screen 100 has a first display screen 101 that displays VAR mode setting information and a second display screen 102 that displays MAX mode setting information.

The first display screen 101 has a mode information display section 103, an output level display section 104 and an output level changing section 105.

The mode information display section 103 displays "VAR" in correspondence with the VAR mode. The output level display section 104 displays the current output level, and a +button 105a and a -button 105b of the output level changing section 105 enable the output level to be changed.

The handpiece 2b has no mode for simultaneously outputting ultrasound and high frequency, and therefore there is no dissection speed display section 86 shown in Fig. 7.

The second display screen 102 has a mode information display section 106 and an output level display section 107.

The mode information display section 106 displays "MAX" in correspondence with the MAX mode. Since this MAX mode is a mode in which ultrasound is outputted at maximum output, that is, level 3 all the time, the output level display section 107 displays level 3. Furthermore, since the output level is level 3 all the time, there is no output level changing section 89 in Fig. 7.

Furthermore, the individual device setting screen 100 has a model name display section 108, a symbol icon 109, FSW connection detection icons 110a and 110b, a communication connection detection icon 111 and a return button 112.

The model name display section 108 displays, for example, "SONICBEAT" (registered trademark) as the model name of the handpiece 2b connected to the ultrasound output apparatus 4. Furthermore, the symbol icon 109 displays the same symbol as the aforementioned symbol 65b as an icon.

The FSW connection detection icons 110a and 110b, the communication connection detection icon 111 and the return button 112 are similar to the FSW connection detection icons 92a and 92b, the communication connection detection icon 93 and the return button 94 respectively, and therefore descriptions thereof will be omitted.

Next, a display screen when the handpieces 2a and 2b are connected to the ultrasound output apparatus 4 will be described.

Fig. 9 is a diagram illustrating an example of display screen when the handpieces 2a and 2b are connected to the ultrasound output apparatus 4. In Fig. 9, the same components as those in Fig. 7 and Fig. 8 will be assigned the same reference numerals and descriptions thereof will be omitted.

The display screen shown in Fig. 9 is a total device display screen 120 which is automatically displayed on the display section when the handpieces 2a and 2b are connected to the ultrasound output apparatus 4.

The total device display screen 120 has a first display screen 121 that displays setting information of the handpiece 2a and a second display screen 122 that displays setting information of the handpiece 2b.

The first display screen 121 has a mode information display section 83, an output level display section 84, a mode information display section 87, an output level display section 88, a model name display section 90, a symbol icon 91, and FSW connection detection icons 92a and 92b.

Furthermore, the second display screen 122 has a mode information display section 103, an output level display section 104, a mode information display section 106, an output level display section 107, a model name display section 108, a symbol icon 109, and FSW connection detection icons 110a and 110b.

As shown in Fig. 9, when the handpieces 2a and 2b are connected to the ultrasound output apparatus 4, the first display screen 121 displays setting information of the handpiece 2a and the second display screen 122 displays setting information of the handpiece 2b.

The screen is partitioned so as to correspond to the arrangement of the output connectors 46a and 46b provided in the ultrasound output apparatus 4. That is, the output connectors 46a and 46b are arranged on the left side and the right side of Fig. 2. Thus, the first display screen 121 and the second display screen 122 are partitioned so as to be arranged on the left side and the right side of Fig. 9.

Furthermore, to make it easier for the user to recognize whether the setting information displayed on the first display screen 121 is setting information of the handpiece 2a or 2b, the arrangement of the output connectors 46a and 46b are designed to match the arrangement of the first display screen 121 and the second display screen 122. That is, the setting information of the handpiece 2a connected to the output connector 46a arranged on the left side of Fig. 2 is displayed on the left side of the total device display screen 120 in Fig. 9. Likewise, the setting information of the handpiece 2b connected to the output connector 46b arranged on the right side of Fig. 2 is displayed on the right side of the total device display screen 120 in Fig. 9. This makes it easier for the user to recognize, for example, whether the setting information displayed on the first display screen 121 is setting information of the handpiece 2a or 2b.

This causes an optimum screen to be automatically displayed according to the device connection situation, and thereby allows the user to see only necessary information, having an effect of improving recognition characteristics of information.

Next, processing of graying out the display screen according to the connection situation will be described.

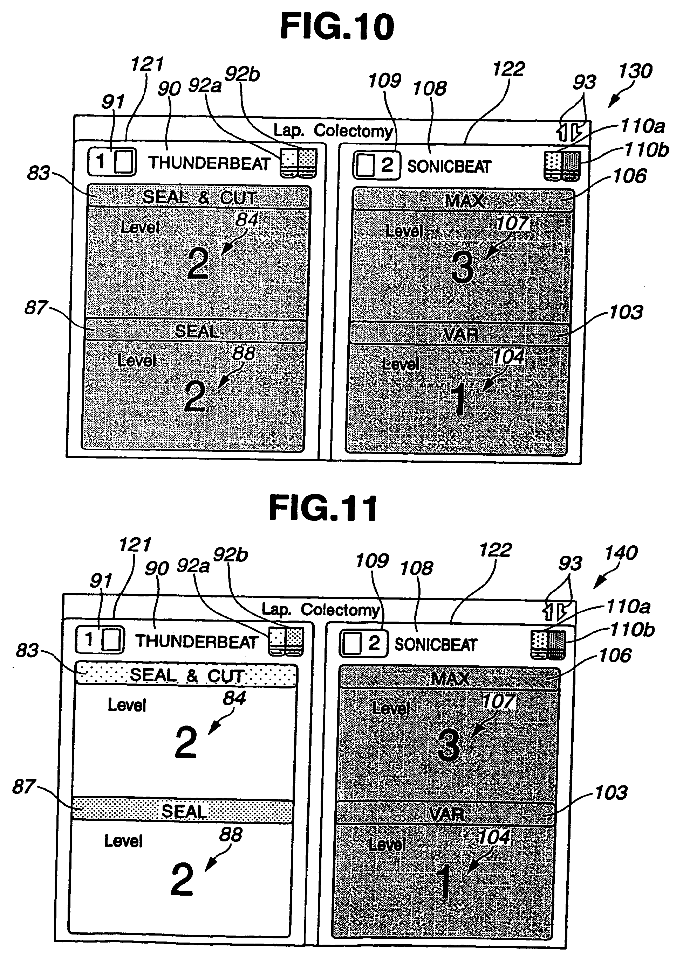

Fig. 10 is a diagram illustrating an example of display screen when the handpieces 2a and 2b are not connected to the ultrasound output apparatus 4. In Fig. 10, the same components as those in Fig. 9 will be assigned the same reference numerals and descriptions thereof will be omitted.

The display screen shown in Fig. 10 is a total device display screen 130 which is automatically displayed on the display section when the handpieces 2a and 2b are not connected to the ultrasound output apparatus 4.

As shown in Fig. 10, when the handpiece 2a is not connected to the ultrasound output apparatus 4, a mode information display section 83, an output level display section 84, a mode information display section 87 and an output level display section 88 of a first display screen 121 are grayed out. Furthermore, the grayed-out region is displayed in a light gray color to allow the output level displayed on the output level display section 84 or the like to be recognized. Likewise, when the handpiece 2b is not connected to the ultrasound output apparatus 4, a mode information display section 103, an output level display section 104, a mode information display section 106 and an output level display section 107 of a second display screen 122 are grayed out.

Here, the user can cause the individual device setting screen 80 shown in Fig. 7 to be displayed by pressing the first display screen 121. The user operates the output level changing sections 85 and 89 of the individual device setting screen 80, and can thereby change the output level of the SEAL & CUT mode and the SEAL mode respectively. After changing the output level, the user can cause the total device display screen 130 to be displayed by pressing the return button 94. Likewise, the user can cause the individual device setting screen 90 shown in Fig. 8 to be displayed by pressing the display screen 122 and cause the total device display screen 130 to be displayed by pressing the return button 112 after changing the output level.

Fig. 11 is a diagram illustrating an example of display screen when the handpiece 2b is not connected to the ultrasound output apparatus 4. In Fig. 11, the same components as those in Fig. 10 will be assigned the same reference numerals and descriptions thereof will be omitted.

The individual device setting screen 80 is automatically displayed when the handpiece 2a is connected to the ultrasound output apparatus 4 and the handpiece 2b is not connected to the ultrasound output apparatus 4, and the display screen shown in Fig. 11 is a total device display screen 140 which is displayed by pressing the return button 94 of the individual device setting screen 80.

As shown in Fig. 11, when the handpiece 2b is not connected to the ultrasound output apparatus 4, a mode information display section 103, an output level display section 104, a mode information display section 106 and an output level display section 107 of the second display screen 122 are grayed out. On the other hand, the first display screen 121 is not grayed out, but remains to be a normal display screen.

Thus, the total device display screen makes it possible to gray out the setting information of the display screen corresponding to the unconnected handpiece, thus make clear the difference between a connection status and a disconnection status and improve recognition characteristics of setting information of the connected handpiece. Furthermore, it is possible to confirm the set value even from a grayed-out status, change the setting of the output level from the individual device setting screen, and change the setting without placing limitations on the operation due to the device connection status, and has therefore an effect of providing a high degree of ease of use.

Next, the processing of highlighting the display screen when the handpiece 2a or 2b is connected to the ultrasound output apparatus 4 will be described.

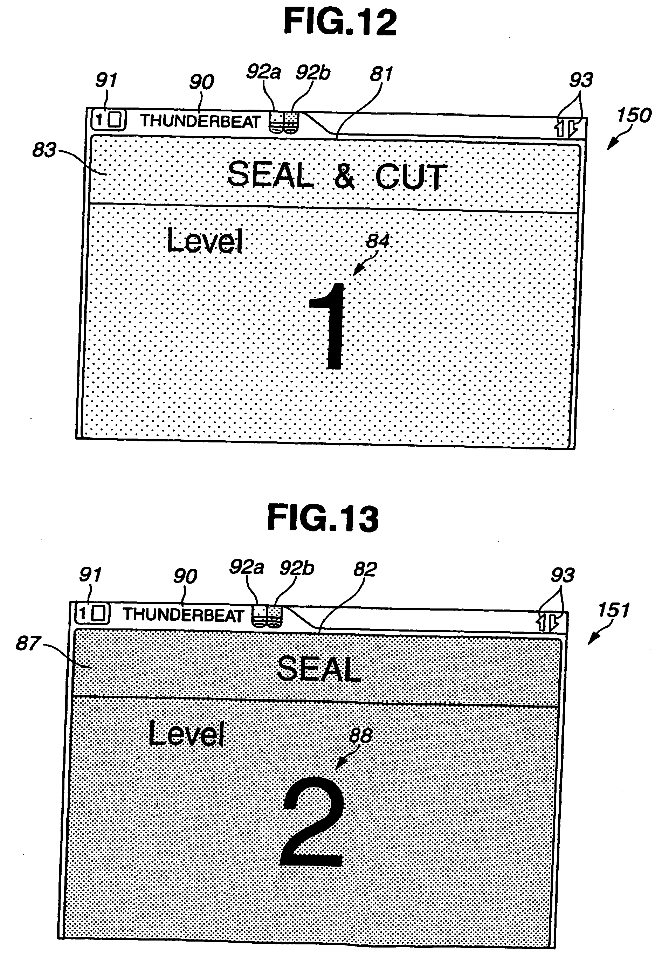

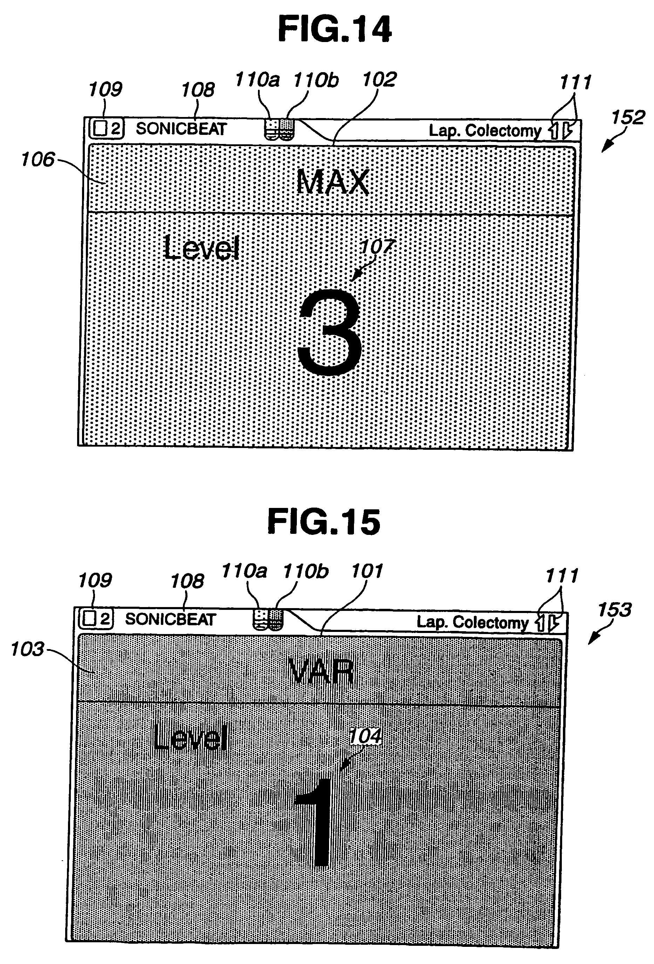

Fig. 12 is a diagram illustrating an example of display screen in the case of output in the SEAL & CUT mode while the handpiece 2a is connected to the ultrasound output apparatus 4, Fig. 13 is a diagram illustrating an example of display screen in the case of output in the SEAL mode while the handpiece 2a is connected to the ultrasound output apparatus 4, Fig. 14 is a diagram illustrating an example of display screen in the case of output in the MAX mode while the handpiece 2b is connected to the ultrasound output apparatus 4, and Fig. 15 is a diagram illustrating an example of display screen in the case of output in the VAR mode while the handpiece 2b is connected to the ultrasound output apparatus 4.

The display screen shown in Fig. 12 is an output mode screen 150 displayed when output is started in the SEAL & CUT mode while the handpiece 2a is connected to the ultrasound output apparatus 4.

When the handpiece 2a is connected to the ultrasound output apparatus 4, the individual device setting screen 80 shown in Fig. 7 is displayed. Here, when output is started in the SEAL & CUT mode, the output mode screen 150 displays setting information of the SEAL & CUT mode on the entire screen. In the output mode screen 150, recognition characteristics of the output mode are improved by performing highlighting covering the entire screen in the aforementioned mode color.

Likewise, the display screen shown in Fig. 13 is an output mode screen 151 displayed when output is started in the SEAL mode while the handpiece 2a is connected to the ultrasound output apparatus 4, the display screen shown in Fig. 14 is an output mode screen 152 displayed when output is started in the MAX mode while the handpiece 2b is connected to the ultrasound output apparatus 4, the display screen shown in Fig. 15 is an output mode screen 153 displayed when output is started in the VAR mode while the handpiece 2b is connected to the ultrasound output apparatus 4.

The output mode screens 151 to 153 also perform highlighting that covers the entire screen in the corresponding mode colors as with the output mode screen 150.

Next, processing of highlighting the display screen while the handpieces 2a and 2b are connected to the ultrasound output apparatus 4 will be described.

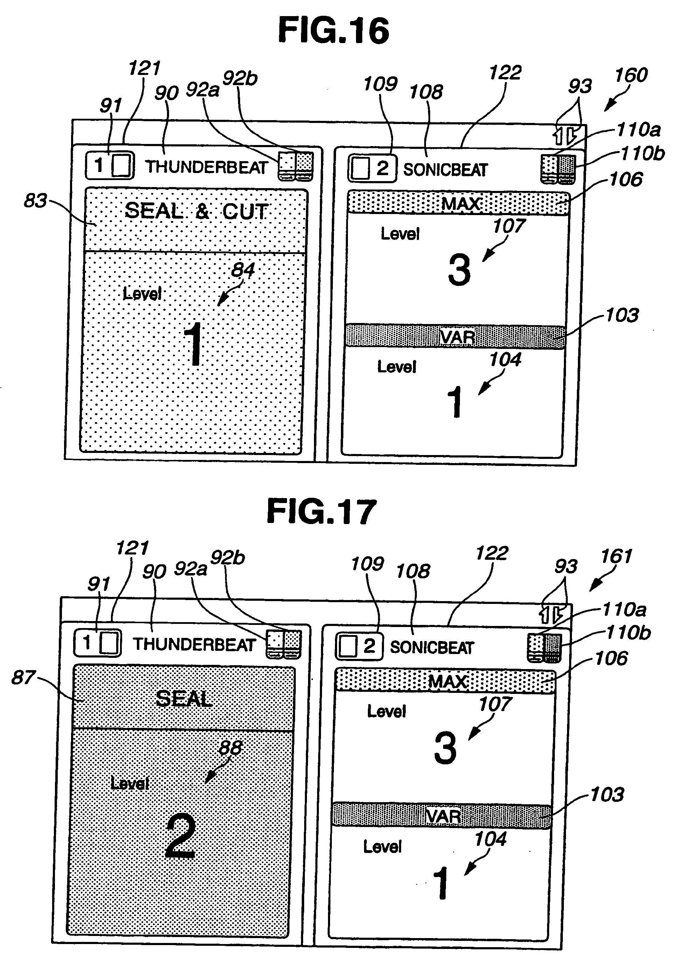

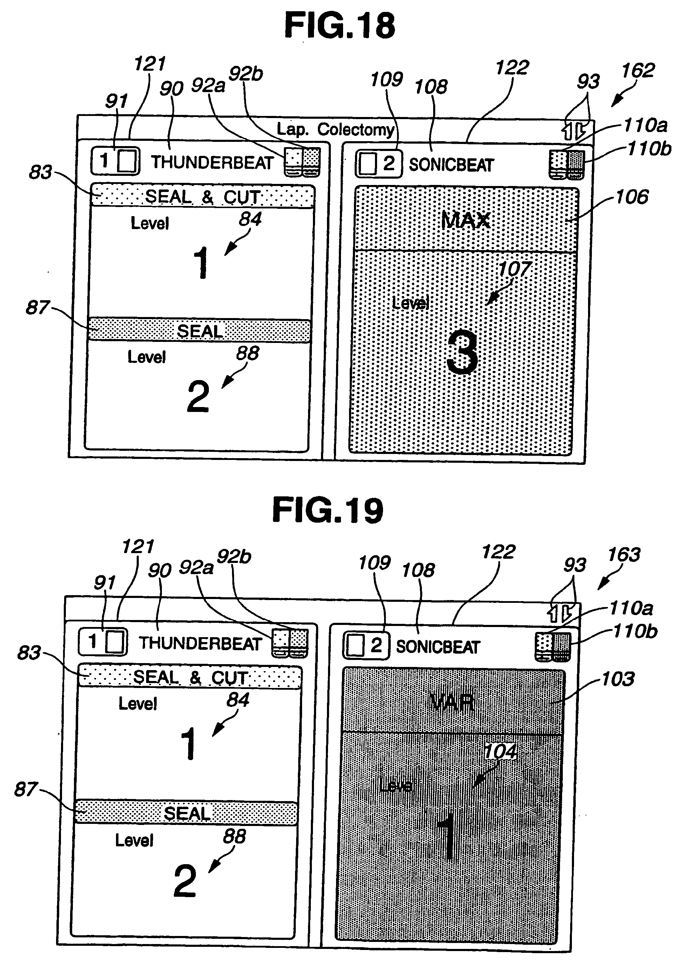

Fig. 16 is a diagram illustrating an example of display screen in the case of output in the SEAL & CUT mode while the handpieces 2a and 2b are connected to the ultrasound output apparatus 4, Fig. 17 is a diagram illustrating an example of display screen in the case of output in the SEAL mode while the handpieces 2a and 2b are connected to the ultrasound output apparatus 4, Fig. 18 is a diagram illustrating an example of display screen in the case of output in the MAX mode while the handpieces 2a and 2b are connected to the ultrasound output apparatus 4 and Fig. 19 is a diagram illustrating an example of display screen in the case of output in the VAR mode while the handpieces 2a and 2b are connected to the ultrasound output apparatus 4.

The display screen shown in Fig. 16 is a total device display screen 160 displayed when output is started in the SEAL & CUT mode while the handpieces 2a and 2b are connected to the ultrasound output apparatus 4.

While the handpieces 2a and 2b are connected to the ultrasound output apparatus 4, the total device display screen 120 shown in Fig. 9 is displayed. Here, when output is started in the SEAL & CUT mode, the total device display screen 160 displays setting information of the SEAL & CUT mode on the entire first display screen 121. By performing highlighting covering the entire first display screen 121 in the aforementioned mode color, the total device display screen 160 improves recognition characteristics of the output mode.

Furthermore, in the total device display screen 120 shown in Fig. 9, pressing the first display screen 121 causes the individual device setting screen 80 shown in Fig. 7 to be displayed. Here, when output in the SEAL & CUT mode is started after operating the + button 85a or - button 85b of the output level changing section 85 and changing the output level, the screen automatically changes to the total device display screen 160 in Fig. 16 and performs highlighting. Thus, even when output is started from the individual device setting screen 80, the screen automatically changes to the total device display screen 160, and the user can thereby intuitively recognize from which of the handpiece 2a or 2b the output originates.

Furthermore, a total device display screen 161 shown in Fig. 17 is displayed when output is started in the SEAL mode while the individual device setting screen 80 in Fig. 7 or the total device display screen 120 in Fig. 9 is displayed.

On the other hand, a total device display screen 162 shown in Fig. 18 is displayed when output is started in the MAX mode while the individual device setting screen 100 in Fig. 8 or the total device display screen 120 in Fig. 9 is displayed.

Furthermore, a total device display screen 163 shown in Fig. 19 is displayed when output is started in the VAR mode while the individual device setting screen 100 in Fig. 8 or the total device display screen 120 in Fig. 9 is displayed.

This allows the user to intuitively recognize the handpiece and the output mode used.

A configuration in which the two handpieces 2a and 2b are connected to the ultrasound output apparatus 4 has been described, but three or more handpieces may be connected to the ultrasound output apparatus 4.

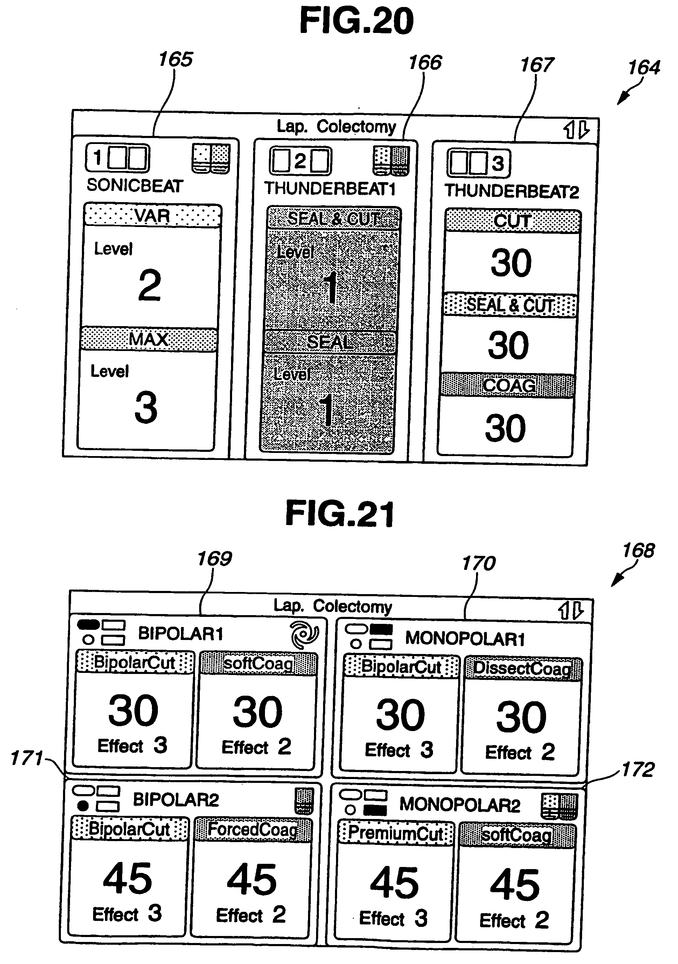

Fig. 20 is a diagram illustrating an example of display screen when three handpieces are connected to the ultrasound output apparatus 4.

The display screen shown in Fig. 20 is a total device display screen 164 which is automatically displayed on the display section while three handpieces are connected to the ultrasound output apparatus 4.

The total device display screen 164 has a first display screen 165, a second display screen 166 and a third display screen 167. Furthermore, the second display screen 166 is grayed out indicating that the corresponding handpiece is not connected. Furthermore, the array of the first to third display screens 165 to 167 matches the array of output connectors provided in the ultrasound output apparatus 4.

Fig. 21 is a diagram illustrating an example of display screen when four handpieces are connected to the ultrasound output apparatus 4.

The display screen shown in Fig. 21 is a total device display screen 168 which is automatically displayed on the display section when four handpieces are connected to the ultrasound output apparatus 4.

The total device display screen 168 has a first display screen 169, a second display screen 170, a third display screen 171 and a fourth display screen 172. Furthermore, the array of the first to fourth display screens 169 to 172 matches the array of output connectors provided in the ultrasound output apparatus 4.

Here, the display processing executed by the GUI control section 57 will be described.

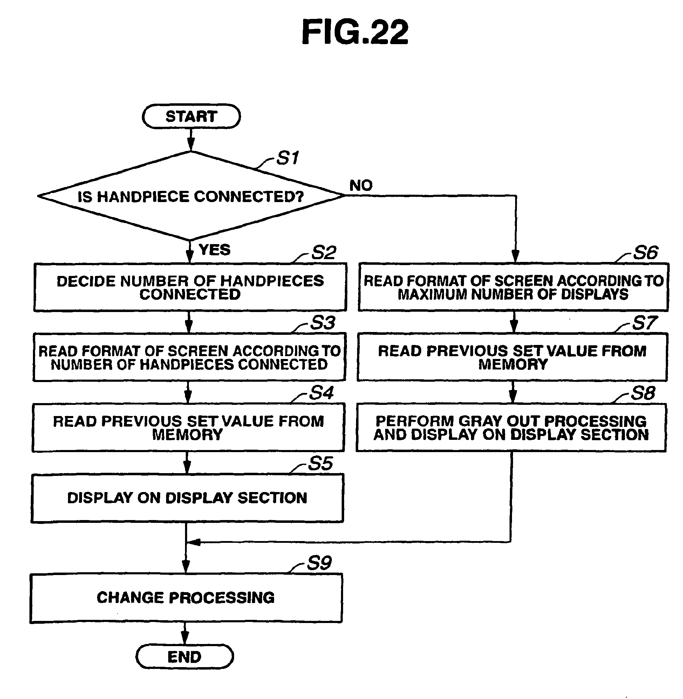

Fig. 22 is a flowchart illustrating a flow of the display processing.

First, it is detected whether or not the handpiece is connected to the ultrasound output apparatus (step S1). "YES" results when it is detected that the handpiece is connected to the ultrasound output apparatus, the number of handpieces connected is detected (step S2) and the format of the screen corresponding to the number of handpieces connected is read (step S3). The previous set value is read from the memory (step S4) and displayed on the display section (step S5).

On the other hand, "NO" results when it is detected in step S 1 that the handpiece is not connected to the ultrasound output apparatus, the format of the screen corresponding to a maximum display number is read (step S6) and the previous set value is read from the memory (step S7). Gray-out processing is then performed and the processing result is displayed on the display section (step S8). Finally, change processing is performed (step S9) and the processing ends.

Next, the change processing in step S9 will be described.

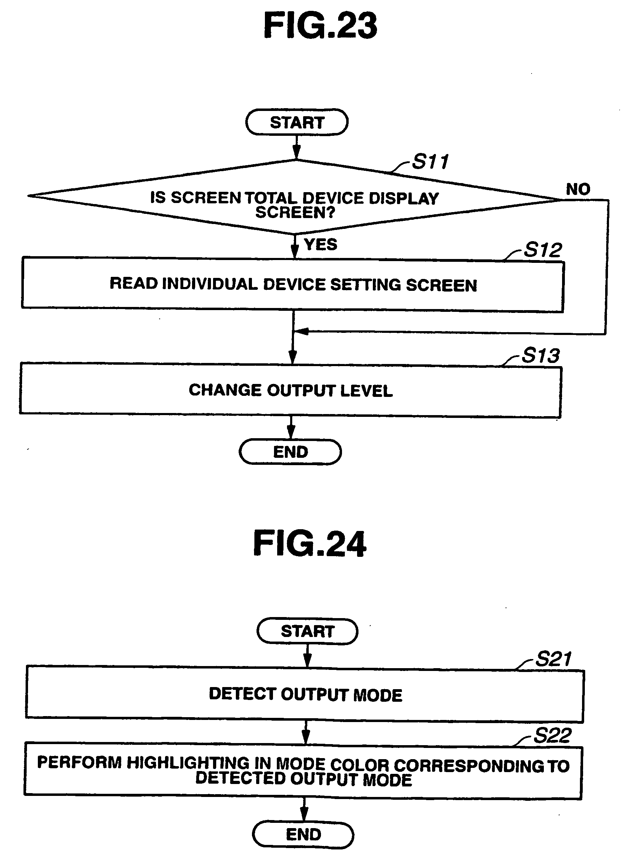

Fig. 23 is a flowchart illustrating an example of flow of the change processing.

First, it is detected whether or not the display screen displayed on the display section is a total device display screen (step S11). "NO" results when the display screen is not the total device display screen and the process moves to step S13. On the other hand, "YES" results when the display screen is the total device display screen and the individual device setting screen is read (step S12). Finally, the output level is changed (step S13) and the processing ends.

Next, the highlighting processing will be described.

Fig. 24 is a flowchart illustrating an example of flow of the highlighting processing.

First, an output mode is detected (step S21). Highlighting is performed in a mode color corresponding to the detected output mode (step S22) and the processing ends.

As described above, the GUI control section 57 of the ultrasound output apparatus 4 performs control so that an optimum display screen is automatically displayed on the display section 60 according to the connection situation of the handpieces 2a and 2b which are medical devices. This allows the user to check only the necessary setting information from the display section 60 and improves recognition characteristics of the setting information.

Furthermore, the GUI control section 57 performs control so that the setting information of the handpiece not connected to the ultrasound output apparatus 4 is displayed grayed out. The user can improve recognition characteristics of the setting information of the handpiece connected.

Furthermore, the GUI control section 57 causes the display screen corresponding to the outputting handpiece to be displayed highlighted in a mode color. This allows the user to intuitively recognize the handpiece and the output mode used.

Steps in the flowcharts of the present specification may be changed in their execution sequence or a plurality of steps may be executed simultaneously or steps may be executed in a sequence which differs every time the steps are executed unless inconsistent with the nature thereof.

The present invention is not limited to the aforementioned embodiment, but various modifications or alterations may be made thereto without departing from the essence of the present invention.

The present application is filed claiming the priority of U.S. Provisional Application No. 61/255,540 filed in U.S. on October 28, 2009.

## Claims

1. An output control apparatus (4) of a medical device to which a first medical device (2a) having at least two output modes for performing treatment of a living tissue and a second medical device (2b) having at least two output modes for performing treatment of the living tissue are connectable, the output control apparatus (4) comprising:
a first connection connector (46a) for connecting the first medical device (2a);
a second connection connector (46b) for connecting the second medical device (2b);
a connection detection section (51) configured to detect whether or not the first medical device (2a) is connected to the first connection connector (46a), and whether or not the second medical device (2b) is connected to the second connection connector (46b);
a display section (60) having a display screen for displaying setting information including mode information corresponding to the respective output modes of the first medical device (2a) and/or setting information including mode information corresponding to the respective output modes of the second medical device (2b); and further
a control section (57) configured to control display of the display section (60) to be a first state, when the connection detection section (51) detects that the first medical device (2a) and the first connection connector (46a) are connected and the second medical device (2b) and the second connection connector (46b) are connected, in which the display screen of the display section (60) is divided into a first display screen (121) for displaying the setting information including the respective mode information of the first medical device and a second display screen (122) for displaying setting information including the respective mode information of the second medical device; to be a second state, when an output of the first medical device is switched to a specified output mode by an operation of a hand switch provided in a grasping portion (6) of the first medical device (2a), in which the mode information corresponding to the specified output mode is displayed to be larger than the display of the mode information in the first state within the first display screen (121); and to be a third state, when an output of the second medical device is switched to a specified output mode by an operation of a hand switch provided in a grasping portion (6) of the second medical device (2b), in which the mode information corresponding to the specified output mode is displayed to be larger than the display of the mode information in the first state within the second display screen (122).

2. The output control apparatus (4) of a medical device according to claim 1, wherein the control section (57) is configured, in the first state, to make division into the first display screen (121) and the second display screen (122) on upper and lower sides or on right and left sides based on an arranging direction of the second connection connector (46b) with respect to the first connection connector (46a), and control display states of the first display screen (121) and the second display screen (122) such that an arrangement relation between the divided first display screen (121) and second display screen (122) matches an arrangement relation between the first connection connector (46a) and the second connection connector (46b).

3. The output control apparatus (4) of a medical device according to claim 1, wherein the control section (57) is configured, when the connection detection section detects that only the first medical device (2a) is connected to the first connection connector (46a), to display the setting information including the respective mode information of the first medical device (2a) on an entire display screen of the display section (60), and, further, when an output is made to the first medical device (2a) in a specified output mode, display the mode information corresponding to the specified output mode to be enlarged on the entire display screen.

4. The output control apparatus (4) of a medical device according to claim 1, wherein the control section (57) is configured, when the connection detection section detects that only the second medical device (2b) is connected to the second connection connector (46b), to display the setting information including the respective mode information of the second medical device (2b) on an entire display screen of the display section (60), and, further, when an output is made to the second medical device (2b) in a specified output mode, display the mode information corresponding to the specified output mode to be enlarged on the entire display screen.

## Patentansprüche

1. Ausgabesteuereinrichtung (4) einer medizinischen Vorrichtung, an die eine erste medizinische Vorrichtung (2a), die mindestens zwei Ausgabemodi zum Ausführen einer Behandlung eines lebenden Gewebes aufweist, und eine zweite medizinische Vorrichtung (2b), die mindestens zwei Ausgabemodi zum Ausführen einer Behandlung des lebenden Gewebes aufweist, anschließbar sind, wobei die Ausgabesteuereinrichtung (4) aufweist:
eine erste Anschlussbuchse (46a) zum Anschließen der ersten medizinischen Vorrichtung (2a);
eine zweite Anschlussbuchse (46b) zum Anschließen der zweiten medizinischen Vorrichtung (2b);
einen Anschlusserfassungsbereich (51), der dazu ausgebildet ist, zu erfassen, ob die erste medizinische Vorrichtung (2a) an die erste Anschlussbuchse (46a) angeschlossen ist oder nicht, und ob die zweite medizinische Vorrichtung (2b) an die zweite Anschlussbuchse (46b) angeschlossen ist oder nicht;
einen Anzeigebereich (60) mit einem Bildschirm zum Anzeigen von Einstellinformationen, die den jeweiligen Ausgabemodi der ersten medizinischen Vorrichtung (2a) entsprechende Modusinformationen beinhalten, und/oder von Einstellinformationen, die den jeweiligen Ausgabemodi der zweiten medizinischen Vorrichtung (2b) entsprechende Modusinformationen beinhalten; und ferner
einen Steuerbereich (57), der dazu ausgebildet ist, eine Anzeige des Anzeigebereichs (60) so zu steuern, dass sie sich in einem ersten Zustand befindet, wenn der Anschlusserfassungsbereich (51) erfasst, dass die erste medizinische Vorrichtung (2a) und die erste Anschlussbuchse (46a) verbunden sind und die zweite medizinische Vorrichtung (2b) und die zweite Anschlussbuchse (46b) verbunden sind, wobei der Bildschirm des Anzeigebereichs (60) in einen ersten Bildschirm (121) zum Anzeigen der die jeweiligen Modusinformationen der ersten medizinische Vorrichtung beinhaltenden Einstellinformationen und einen zweiten Bildschirm (122) zum Anzeigen von die jeweiligen Modusinformationen der zweiten medizinischen Vorrichtung beinhaltenden Einstellinformationen unterteilt ist; dass sie sich in einem zweiten Zustand befindet, wenn eine Ausgabe der ersten medizinischen Vorrichtung durch Betätigung eines in einem Griffbereich (6) der ersten medizinischen Vorrichtung (2a) vorgesehenen Handschalters in einen festgelegten Ausgabemodus geschaltet wird, wobei die dem festgelegten Ausgabemodus entsprechenden Modusinformationen so angezeigt werden, dass sie größer sind als die Anzeige der Modusinformationen in dem ersten Zustand innerhalb des ersten Bildschirms (121); und dass sie sich in einem dritten Zustand befindet, wenn eine Ausgabe der zweiten medizinischen Vorrichtung durch Betätigung eines in einem Griffbereich (6) der zweiten medizinischen Vorrichtung (2b) vorgesehenen Handschalters in einen festgelegten Ausgabemodus geschaltet wird, wobei die dem festgelegten Ausgabemodus entsprechenden Modusinformationen so angezeigt werden, dass sie größer sind als die Anzeige der Modusinformationen in dem ersten Zustand innerhalb des zweiten Bildschirms (122).

2. Ausgabesteuereinrichtung (4) einer medizinischen Vorrichtung nach Anspruch 1, wobei der Steuerbereich (57) dazu ausgebildet ist, auf der Grundlage einer Anordnungsrichtung der zweiten Anschlussbuchse (46b) bezüglich der ersten Anschlussbuchse (46a), in dem ersten Zustand eine Unterteilung in den ersten Bildschirm (121) und in den zweiten Bildschirm (122) auf der oberen und unteren Seite oder auf der rechten und linken Seite vorzunehmen, und den jeweiligen Anzeigezustand des ersten Bildschirms (121) und des zweiten Bildschirms (122) so zu steuern, dass eine Anordnungsbeziehung zwischen dem unterteilten ersten Bildschirm (121) und zweiten Bildschirm (122) mit einer Anordnungsbeziehung zwischen der ersten Anschlussbuchse (46a) und der zweiten Anschlussbuchse (46b) übereinstimmt.

3. Ausgabesteuereinrichtung (4) einer medizinischen Vorrichtung nach Anspruch 1, wobei der Steuerbereich (57) dazu ausgebildet ist, die Einstellinformationen einschließlich der jeweiligen Modusinformationen der ersten medizinischen Vorrichtung (2a) auf einem gesamten Bildschirm des Anzeigebereichs (60) anzuzeigen, wenn der Anschlusserfassungsbereich erfasst, dass nur die erste medizinische Vorrichtung (2a) an der ersten Anschlussbuchse (46a) angeschlossen ist, und ferner, wenn eine Ausgabe an die erste medizinische Vorrichtung (2a) in einem festgelegten Ausgabemodus erfolgt, die dem festgelegten Ausgabemodus entsprechenden Modusinformationen in einer auf den gesamten Bildschirm vergrößerten Weise anzuzeigen.

4. Ausgabesteuereinrichtung (4) einer medizinischen Vorrichtung nach Anspruch 1, wobei der Steuerbereich (57) dazu ausgebildet ist, die Einstellinformationen einschließlich der jeweiligen Modusinformationen der zweiten medizinischen Vorrichtung (2b) auf einem gesamten Bildschirm des Anzeigebereichs (60) anzuzeigen, wenn der Anschlusserfassungsbereich erfasst, dass nur die zweite medizinische Vorrichtung (2b) an der zweiten Anschlussbuchse (46b) angeschlossen ist, und ferner, wenn eine Ausgabe an die zweite medizinische Vorrichtung (2b) in einem festgelegten Ausgabemodus erfolgt, die dem festgelegten Ausgabemodus entsprechenden Modusinformationen in einer auf den gesamten Bildschirm vergrößerten Weise anzuzeigen.

## Revendications

1. Appareil de commande de sortie (4) d'un dispositif médical auquel un premier dispositif médical (2a) ayant au moins deux modes de sortie destinés à exécuter un traitement d'un tissu vivant et un deuxième dispositif médical (2b) ayant au moins deux modes de sortie destinés à réaliser un traitement du tissu vivant peuvent être connectés, l'appareil de commande de sortie (4) comprenant :
un premier connecteur de raccordement (46a) destiné à raccorder le premier dispositif médical (2a) ;
un deuxième connecteur de raccordement (46b) destiné à raccorder le deuxième dispositif médical (2b) ;
une section de détection de raccordement (51) configurée pour détecter si ou non le premier dispositif médical (2a) est raccordé au premier connecteur de raccordement (46a), et si oui ou non le deuxième dispositif médical (2b) est raccordé au deuxième connecteur de raccordement (46b) ;
une section d'affichage (60) comportant un écran d'affichage destiné à afficher des informations de réglage incluant des informations de mode correspondant aux modes de sortie respectifs du premier dispositif médical (2a) et/ou des informations de réglage incluant des informations de mode correspondant aux modes de sortie respectifs de deuxième dispositif médical (2b) ; et de plus
une section de commande (57) configurée pour commander l'affichage de la section d'affichage (60) pour se trouver dans un premier état, lorsque la section de détection de raccordement (51) détecte que le premier dispositif médical (2a) et le premier connecteur de raccordement (46a) sont raccordés et que le deuxième dispositif médical (2b) et le deuxième connecteur de raccordement (46b) sont raccordés, dans laquelle l'écran d'affichage de la section d'affichage (60) est divisé en un premier écran d'affichage (121) destiné à afficher les informations de réglage incluant les informations de mode respectif du premier dispositif médical et un deuxième écran d'affichage (122) destiné à afficher des informations de réglage incluant les informations de mode respectif du deuxième dispositif médical ; pour se trouver dans un deuxième état, lorsqu'une sortie du premier dispositif médical est commutée vers un mode de sortie spécifié par un actionnement d'un interrupteur à main prévu dans une partie de préhension (6) du premier dispositif médical (2a), dans lequel les informations de mode correspondant au mode de sortie spécifié sont affichées pour être plus grandes que l'affichage des informations de mode dans le premier état à l'intérieur du premier écran d'affichage (121) ; et pour se trouver dans un troisième état, lorsqu'une sortie du deuxième dispositif médical est commutée vers un mode de sortie spécifié par un actionnement d'un interrupteur à main prévu dans une partie de préhension (6) du deuxième dispositif médical (2b), dans lequel les informations de mode correspondant au mode de sortie spécifié sont affichées pour être plus grandes que l'affichage des informations de mode dans le premier état à l'intérieur du deuxième écran d'affichage (122).

2. Appareil de commande de sortie (4) d'un dispositif médical selon la revendication 1, dans lequel la section de commande (57) est configurée, dans le premier état, pour effectuer une division en le premier écran d'affichage (121) et le deuxième écran d'affichage (122) sur les côtés supérieur et inférieur ou sur les côtés droit et gauche sur la base d'une direction d'agencement du deuxième connecteur de raccordement (46b) par rapport au premier connecteur de raccordement (46a), et commande les états d'affichage du premier écran d'affichage (121) et du deuxième écran d'affichage (122) d'une manière telle qu'une relation d'agencement entre le premier écran d'affichage (121) et le deuxième écran d'affichage (122) divisés correspond à une relation d'agencement entre le premier connecteur de raccordement (46a) et le deuxième connecteur de raccordement (46b).

3. Appareil de commande de sortie (4) d'un dispositif médical selon la revendication 1, dans lequel la section de commande (57) est configurée, lorsque la section de détection de raccordement détecte que seul le premier dispositif médical (2a) est raccordé au premier connecteur de raccordement (46a), pour afficher les informations de réglage incluant les informations de mode respectif du premier dispositif médical (2a) sur un écran d'affichage entier de la section d'affichage (60), et, de plus, lorsqu'une sortie est effectuée vers le premier dispositif médical (2a) dans un mode de sortie spécifié, afficher les informations de mode correspondant au mode de sortie spécifié pour être agrandies sur l'écran d'affichage entier.

4. Appareil de commande de sortie (4) d'un dispositif médical selon la revendication 1, dans lequel la section de commande (57) est configurée, lorsque la section de détection de raccordement détecte que seul le deuxième dispositif médical (2b) est raccordé au deuxième connecteur de raccordement (46b), pour afficher les informations de réglage incluant les informations de mode respectif du deuxième dispositif médical (2b) sur un écran d'affichage entier de la section d'affichage (60), et, de plus, lorsqu'une sortie est effectuée vers le deuxième dispositif médical (2b) dans un mode de sortie spécifié, afficher les informations de mode correspondant au mode de sortie spécifié pour être agrandies sur l'écran d'affichage entier.
